# FASCICULE DE BREVET EUROPEEN

(11) **EP 2 300 043 B1**
(45) Date de publication et mention de la délivrance du brevet: **17.10.2012**
(21) Numéro de dépôt: 09757702.7
(22) Date de dépôt: 04.06.2009
(51) Int. Cl.: A61K 38/16, A61P 29/00, A61P 17/04

(54) **EFFETS ANALGESIQUES DE LA TOXINE PEPTIDIQUE APETX2**
ANALGETISCHE WIRKUNGEN VON PEPTIDTOXIN APETX2
ANALGESIC EFFECTS OF PEPTIDE TOXIN APETX2

(30) Priorité: 06.06.2008 FR 0803158
(43) Date de publication de la demande: 30.03.2011
(73) Titulaire: Centre National de la Recherche Scientifique - CNRS, 75794 Paris Cedex 16 (FR); Université Nice Sophia Antipolis, 06108 Nice Cedex 2 (FR)
(72) Inventeur: DEVAL , Emmanuel, F-06220 Golfe Juan (FR); DIOCHOT, Sylvie, F-06560 Valbonne (FR); LAZDUNSKI, Michel, F-06000 Nice (FR); LINGUEGLIA, Eric, F-06000 Nice (FR); NOEL, Jacques, F-06100 Nice (FR)
(74) Mandataire: Patarin, Stephanie
(86) Numéro de dépôt international: PCT/FR2009/000657
(87) Numéro de publication internationale: WO 2009/147326

(56) Documents cités:
- US-A1- 2008 181 881
- DEVAL EMMANUEL ET AL: "ASIC3, a sensor of acidic and primary inflammatory pain" EMBO (EUROPEAN MOLECULAR BIOLOGY ORGANIZATION) JOURNAL, vol. 27, no. 22, novembre 2008 (2008-11), pages 3047-3055, XP009110553 ISSN: 0261-4189
- CHEN CHIH-CHENG: "ASIC3 and muscle pain" NOVEL TRENDS IN BRAIN SCIENCE:BRAIN IMAGING, LEARNING AND MEMORY, STRESS AND FEAR, AND PAIN SPRINGER, 233 SPRING STREET, NEW YORK, NY 10013, UNITED STATES, 2008, pages 225-232, XP002509971 ISSN: 978-4-431-73241-9(H)
- DIOCHOT S ET AL: "A new sea anemone peptide, APETx2, inhibits ASIC3, a major acid-sensitive channel in sensory neurons" EMBO JOURNAL 20040407 GB, vol. 23, no. 7, 7 avril 2004 (2004-04-07), pages 1516-1525, XP009110554 ISSN: 0261-4189 cité dans la demande
- CHEN C -C ET AL: "A role for ASIC3 in the modulation of high-intensity pain stimuli" PROCEEDINGS OF THE NATIONAL ACADEMY OF SCIENCES OF THE UNITED STATES OF AMERICA 20020625 US, vol. 99, no. 13, 25 juin 2002 (2002-06-25), pages 8992-8997, XP002509972 ISSN: 0027-8424
- TOMOHIRO HONMA ET AL: "Peptide Toxins in Sea Anemones: Structural and Functional Aspects" MARINE BIOTECHNOLOGY, SPRINGER-VERLAG, NE, vol. 8, no. 1, 1 janvier 2006 (2006-01-01), pages 1-10, XP019368183 ISSN: 1436-2236
- MOLLIVER DEREK C ET AL: "ASIC3, an acid-sensing ion channel, is expressed in metaboreceptive sensory neurons" MOLECULAR PAIN, BIOMED CENTRAL, LONDON, GB, vol. 1, no. 1, 23 novembre 2005 (2005-11-23), page 35, XP021011227 ISSN: 1744-8069
- VOILLEY N ET AL: "Nonsteroid anti-inflammatory drugs inhibit both the activity and the inflammation-induced expression of acid-sensing ion channels in nociceptors" JOURNAL OF NEUROSCIENCE 20011015 US, vol. 21, no. 20, 15 octobre 2001 (2001-10-15), pages 8026-8033, XP002509973 ISSN: 0270-6474
- DIOCHOT ET AL: "Peptides inhibitors of acid-sensing ion channels" TOXICON, ELMSFORD, NY, US, vol. 49, no. 2, 26 janvier 2007 (2007-01-26), pages 271-284, XP005738071 ISSN: 0041-0101
- CHAGOT BENJAMIN ET AL: "Solution structure of APETx2, a specific peptide inhibitor of ASIC3 proton-gated channels" PROTEIN SCIENCE, CAMBRIDGE UNIVERSITY PRESS, CAMBRIDGE, GB, vol. 14, no. 8, 29 juin 2005 (2005-06-29), pages 2003-2010, XP009110558 ISSN: 0961-8368

### SEQUENCE LISTING

<110> CENTRE NATIONAL DE LA RECHERCHE SCIENTIFIQUE - CNRS
   DEVAL, Emmanuel
   DIOCHOT, Sylvie
   LAZDUNSKI, Michel
   LINGUEGLIA, Eric
   NOEL, Jacques
<120> EFFETS ANALGESIQUES DE LA TOXINE PEPTIDIQUE APETx2
<130> 104025/PCT
<150> FR 08/03158
   <151> 2008-06-06
<160> 2
<170> PatentIn version 3.5
<210> 1
   <211> 19
   <212> DNA
   <213> Artificial Sequence
<220>
   <223> ARNi spécifique des canaux ASIC3
<400> 1
   ctacacgcta tgccaagga 19
<210> 2
   <211> 19
   <212> DNA
   <213> Artificial Sequence
<220>
   <223> ARNi contrôle
<400> 2
   gctcacacta cgcagagat 19

## Description

### Domaine technique de l'invention

L'invention se rapporte à l'utilisation de la toxine peptidique APETx2 inhibitrice des canaux cationiques ASIC3 issue de l'anémone de mer *Anthopleura elegantissima,* pour ses effets analgésiques vis-à-vis de la douleur associée à l'activation de l'isoforme ASIC3 (Acid Sensing Ion Channel 3) lors d'une inflammation, et aussi potentiellement lors de toutes situations douloureuses associées avec une acidose tissulaire (ischémies, fractures, hématomes, oedèmes, phlyctènes, infections locales, lésions tissulaires, blessures oculaires, tumeurs, etc...)..

### Etat de la technique antérieure

La prise en compte et le traitement des douleurs, notamment inflammatoires, sont des aspects essentiels de l'amélioration de la qualité de vie des patients, et repose essentiellement sur la prescription d'anti-inflammatoires, qu'ils soient non-stéroïdiens (AINS) ou stéroïdiens. Lorsque les AINS et/ou les corticoïdes ne suffisent pas à soulager une douleur inflammatoire, le prescripteur associe un antalgique non anti-inflammatoire, par exemple du paracétamol, avec des opioïdes faibles ou forts. Cependant malgré la diversité de l'arsenal thérapeutique existant, de nombreuses douleurs restent peu sensibles aux drogues connues qui peuvent de plus générer des effets secondaires indésirables, comme dans le cas des AINS. Dans ce contexte, la découverte de nouvelles cibles analgésiques représenterait donc un réel progrès. Parmi les cibles moléculaires identifiées ces dernières années, les canaux ioniques tiennent une place particulièrement importante car ils sont directement impliqués dans la détection et la transmission de signaux douloureux par les fibres nociceptives.

Les ASICs (Acid Sensing Ion Channels) sont des canaux cationiques activés par une acidose extracellulaire (pour revue, [réf. 1] et [r é f. 2]). Jusqu'à présent, quatre gènes codant pour au moins sept sous-unités (ASIC1a, ASIC1b, ASIC1b2, ASIC2a, ASIC2b, ASIC3 et ASIC4) ont été identifiés chez les mammifères. Des canaux ASICs fonctionnels résultent de l'association de différentes sous-unités ASIC en trimères ([réf. 3]) conduisant à des canaux homomères ou hétéromères ([réf. 4, 5 et 6]). Les canaux ASICs sont en majorité des canaux neuronaux, exprimés à la fois dans les systèmes nerveux central et périphérique. Alors que les canaux ASIC1a et ASIC2 sont largement présents à la fois dans les systèmes nerveux central et périphérique, l'expression des canaux ASIC1b et ASIC3 est restreinte aux neurones sensoriels ([réf. 7, 57 et 8]).

Il a été postulé que les ASICs sont capables de détecter les acidifications extracellulaires susceptibles de se développer lors d'une ischémie, d'une inflammation, d'un hématome, d'une fracture, d'une lésion, d'une intervention chirurgicale (douleur post-opératoire), ou du développement de certaines tumeurs ([réf. 9]). Or on sait depuis plusieurs années maintenant que l'acidose extracellulaire génère de la douleur ([réf. 11 et 12]), et des expérimentations réalisées sur des volontaires humains sains ([réf. 13 et 14]) ont montré l'implication des ASICs dans la douleur cutanée acide à l'aide de l'amiloride et de certains AINS, des inhibiteurs non spécifiques des ASICs ([réf. 15 et 17]).

Parmi toutes les sous-unités ASICs exprimées dans les neurones sensoriels, ASIC3 est d'un intérêt particulier du fait qu'il est largement exprimé dans les neurones nociceptifs ([réf. 7], précitée ;[réf. 17 et 18]), et qu'il génère un courant non-inactivant, persistant en réponse à une acidification modérée (à environ pH 7.0) ([réf. 19]). Le courant d'ASIC3 a en effet deux composantes: (1) une composante transitoire qui est fortement sensible au pH (pH_{0,5} = 6,5 - 6,7) ([réf. 7], précitée ;[réf. 20]) rapidement activant (t_{0.5} < 5ms) et inactivant (t_{0.5} = 0,32s) ([réf. 20], précitée), et (2) une composante soutenue qui provient pour des acidifications modérées (entre pH 7,3 et 6,7) du courant de fenêtre issu du chevauchement partiel des courbes d'activation et d'inactivation du canal ([réf. 19], précitée) et pour des pH plus acides (pH < 6,0) d'un mécanisme apparemment différent. Le courant transitoire récupère rapidement suite à une inactivation après un retour à un pH neutre (t_{0.5} = 0,58s) ([réf. 20], précitée). A titre de comparaison, ASIC1a nécessite un temps de récupération beaucoup plus long (t_{0.5} = 13s) ([réf. 20], précitée). Le courant transitoire s'inactive rapidement lorsque le pH de repos est acide. Inversement, le courant soutenu peut encore être activé lorsque le pH diminue à partir d'un pH de repos relativement acide (< pH 6) et est également activé lorsque le pH extracellulaire diminue graduellement ([réf. 7], précitée). De récentes études chez les souris knock-out proposent un rôle d'ASIC3 dans la détection de l'acidose tissulaire dans les muscles et les articulations dans des modèles d'hyperalgésie mécanique secondaire induite par l'inflammation ou par des injections répétées d'acide dans le muscle ([réf. 21, 22, 23 et 60]). Une implication d'ASIC3 dans la mécanosensibilité des neurones sensoriels de grands diamètres a également été postulée ([réf. 21]). En revanche, la comparaison de souris normales et de souris knock-out n'a pas pu mettre en évidence un rôle significatif d'ASIC3 dans la douleur cutanée acide ou dans l'hypersensibilité cutanée à la douleur associée à une inflammation ([réf. 21], précitée et [réf. 25]).

Aussi, si les études passées permettent au mieux de prédire que l'inhibition de l'activité d'ASIC3 pourrait a *priori* affecter la douleur, l'implication réelle des ASICs et la participation relative des différentes isoformes présentes dans les neurones sensoriels, notamment d'ASIC3, dans la sensibilité *in vivo* des nocicepteurs à l'acide et dans la douleur cutanée acide en conditions normales ou inflammatoires, restent encore à démontrer. En outre, il reste également à démontrer de quelle manière, hyperalgésie ou analgésie, l'inhibition de l'activité des ASICs, notamment d'ASIC3, pourrait affecter la douleur. Or les analyses complémentaires requises nécessitent des outils pharmacologiques sélectifs.

Jusqu'à récemment, le répertoire de ligands actifs capables d'inhiber ASIC3 était principalement limité à l'amiloride et aux drogues anti-inflammatoires non stéroïdiennes (AINS) ([réf. 7], précitée et [réf. 17], précitée). Cependant, aucune de ces drogues n'est absolument spécifique des canaux ASIC ou d'un type de canal ASIC particulier, et notamment d'ASIC3.

Depuis plusieurs années, les venins d'animaux ont produit un grand nombre de toxines capables de moduler spécifiquement avec une grande affinité les courants Ca²⁺, K⁺ et Na⁺ voltage-dépendants ([réf. 28, 29, 30, 31, 32, 33]), les canaux potassiques Ca²⁺-dépendants ([réf. 34 et 35]), et les canaux potassiques mécanosensibles ([réf. 58]). Récemment, deux toxines animales (PcTx1 et APETx2) capables de bloquer spécifiquement les canaux ASIC1a ([réf. 36]) et ASIC3 ([réf. 38]), respectivement, ont été identifiées.

Dans le but d'identifier des effecteurs spécifiques du canal ASIC3, un très grand nombre de venins de scorpion, d'abeille, d'araignée, de serpent et d'anémone de mer (dilution au 1/1000) ou de fractions peptidiques (0,1 mg/ml) a été criblé vis-à-vis de canaux ASIC3 exprimés dans des ovocytes de Xénope. Il a été mis en évidence qu'une fraction peptidique de l'anémone de mer *Anthopleura elegantissima* inhibe plus de 80% du courant de l'ASIC3 de rat stimulé à pH 6. Le peptide actif a été purifié jusqu'à l'homogénéité par chromatographie à polarité de phase inversée et d'échange cationique guidée par un suivi des fractions actives sur ASIC3, et a été dénommé APETx2 ([réf. 38]).

APETx2 est un peptide de 42 acides aminés comprenant trois ponts disulfures, avec une organisation structurale similaire à celle d'autres toxines d'anémone de mer qui bloquent les canaux potassiques et sodiques sensibles au voltage. Sa séquence complète a été établie par dégradation N-terminale d'Edman, et sa masse monoisotopique mesurée (4557,96 Da) est en parfait accord avec sa masse calculée à partir des données de séquence (4557,88 Da, précision à 17,5 ppm), indiquant une extrémité C-terminale libre. APETx2 présente 64% d'identité (76% d'homologie) de séquence avec APETx1 ([réf. 40]) et seulement 34% d'identité de séquence (57 et 55% d'homologie, respectivement) avec les toxines BDS-1 et BDS-II d*'Anemonia sulcata,* qui inhibent le courant potassique Kv3.4 voltage-dépendant ([réf. 41]). L'identité de séquence avec les peptides activateurs des courants sodiques, tels que AP-A, AP-B ; AP-C, APE1-1, et APE-2 *d'Anthopieura sp.* ([réf. 39]), est seulement de 25-29% (homologie de 41-47%). En outre, APETx2 ne présente aucune homologie de séquence avec PcTx1, l'inhibiteur spécifique de l'isoforme ASIC1a ([réf. 36], précitée).

APETx2 bloque directement ASIC3 en agissant sur sa partie externe, et ne modifie pas la conductance unitaire du canal. APETx2 bloque de manière réversible ASIC3 en inhibant le courant transitoire (IC₅₀ = 63 nM) sans affecter le courant soutenu, mais est sans aucun effet sur les isoformes ASIC1a, ASIC1b et ASIC2a. APETx2 inhibe également le courant de l'hétéromère ASIC2b+3, alors qu'il a moins d'affinité pour l'hétéromère ASIC1b+3 et ASICIa+3, et aucun effet sur l'hétéromère ASIC2a+3 ([réf. 38], précitée).

De manière surprenante, les Inventeurs ont maintenant démontré que l'injection périphérique, notamment sous-cutanée, de la toxine peptidique APETx2 permet de diminuer la douleur (effet analgésique) associée à l'activation de l'isoforme ASIC3 dans des modèles de douleur chez le rat liée à une inflammation et à l'injection sous-cutanée de solutions acides mimant toutes situations douloureuses associées à une acidose tissulaire (ischémies, fractures, lésions tissulaires, tumeurs, etc...).

### Exposé de l'invention

L'invention vise à remédier aux inconvénients de l'état de la technique, et notamment à apporter de nouvelles molécules analgésiques spécifiques de la cible, présentant peu ou pas d'effets secondaires indésirables, et faciles d'utilisation, notamment par application périphérique (sous-cutanée, intramusculaire, transcutanée, cutanée, etc...).

L'invention porte sur la toxine peptidique APETx2 de l'anémone de mer *Anthopleura elegantissima,* peptides isolés d'autres venins d'anémone de mer ou d'autres espèces marines de la même famille présentant de 60 à 99% d'identité de séquence avec la séquence de 42 acides aminés de la toxine peptidique APETx2, et peptides isolés du venin de l'anémone de mer *Anthopleura elegantissima* présentant (i) au moins une substitution sur les positions 3, 5, 8, 9, 10, 15-17, 23, 31-33, 36, 39 et 41 ou (ii) une extension d'au moins un acide aminé sur les extrémités N- et/ou C-terminales, de la séquence de 42 acides aminés de la toxine peptidique APETX2, pour une utilisation comme médicament analgésique,
lesdits peptides conservant la propriété de la toxine peptidique APETx2 à inhiber les canaux de type ASIC3.

On entend par « analogues de la toxine peptidique APETx2 », des peptides isolés d'autres venins d'anémone de mer ou d'autres espèces marines de la même famille et présentant la même propriété d'inhibition des canaux de type ASIC3. Par exemple, il s'agit de peptides ayant de 60 à 99 % identité de séquence avec la séquence de 42 acides aminés de la toxine peptidique d'APETx2.

On entend par « dérivés de la toxine peptidique APETx2 », des peptides isolés du même venin qui présentent une délétion et/ou une extension et/ou une substitution d'un ou plusieurs acides aminés dans la séquence de 42 acides aminés de la toxine peptidique APETx2, et qui conservent la propriété d'inhibition des canaux de type ASIC3. Par exemple, il peut s'agir de variants peptidiques présentant une substitution sur les positions 3, 5, 8, 9, 10, 15, 16, 17, 23, 31, 32, 33, 36, 39, et/ou 41 dans la séquence de 42 acides aminés de la toxine peptidique APETx2. Il peut aussi s'agir de variants peptidiques présentant une extension d'un ou plusieurs acides aminés sur les extrémités N- et/ou C-terminales de la séquence de la toxine peptidique APETx2.

Selon un mode de réalisation particulier de l'invention, ledit médicament est destiné à prévenir ou traiter des pathologies impliquant les canaux de type ASIC3. Par exemple, lesdites situations pathologiques sont choisies parmi le groupe constitué des inflammations y compris les gastrites, des ischémies (musculaires, cardiaques, mésentériques...), des fractures, des hématomes, des oedèmes, des phlyctènes (ou ampoules ou cloques), des infections locales, des lésions tissulaires y compris les incisions liées à un acte chirurgical, des blessures oculaires, des prurits et des tumeurs y compris les tumeurs et métastases osseuses.

Ledit médicament est un analgésique, de préférence destiné à la prévention ou au traitement de la douleur induite par une activation des canaux de type ASIC3, plus préférentiellement destiné à la prévention ou au traitement d'une douleur résultant d'une inflammation.

Selon un autre mode de réalisation particulier de l'invention; ledit analgésique est destiné à la prévention ou au traitement de la douleur acide associée à une activation des canaux de type ASIC3. Par exemple, les situations douloureuses associées à une acidose tissulaire sont choisies parmi le groupe constitué des ischémies (musculaires, cardiaques, mésentériques,...), des fractures, des hématomes, des oedèmes, des phlyctènes (ou ampoules ou cloques), des infections locales, des lésions tissulaires y compris les incisions liées à un acte chirurgical (douleurs post-opératoires), des blessures oculaires, et au sein de tumeurs (y compris les tumeurs et métastases osseuses).

Selon encore un autre mode de réalisation de l'invention, ledit analgésique est destiné à la prévention ou au traitement des démangeaisons, dans la mesure où celles-ci proviennent d'un processus pathologique mettant en jeu des voies sensorielles similaires ([réf. 61]) de celles de la douleur associée à l'activation des canaux de type ASIC3.

Selon un mode particulier de réalisation de l'invention, ledit médicament est administré par voie périphérique, par exemple par voie sous-cutanée, intramusculaire, transcutanée ou encore cutanée. De préférence ledit médicament est administré par voie sous-cutanée.

### Brève description des figures

D'autres caractéristiques et avantages de l'invention ressortiront à la lecture de la description qui suit, en référence aux figures annexées :
- la figure 1 est une image en fluorescence d'un neurone cutané des ganglions rachidiens marqué de manière rétrograde et des courants ASIC enregistrés dans ces neurones;
- la figure 2 représente l'effet de la pression osmotique (hypertonicité) et de l'acide arachidonique sur le courant ASIC natif induit par pH 7,0;
- la figure 3 représente la potentialisation par l'hypertonicité des courants ASIC1a et ASIC3 recombinants induits par une acidification modérée;
- la figure 4 représente l'augmentation du courant de fenêtre ASIC3 non-inactivant par le médiateur inflammatoire, l'acide arachidonique (AA) et l'effet synergique sur ce courant de l'acide arachidonique et de l'hypertonicité lors d'une acidose modérée.
- la figure 5 représente le rôle *in vivo* d'ASIC3 dans la douleur cutanée acide, sa modulation positive par les facteurs inflammatoires (acide arachidonique et hypertonicité) et son rôle dans l'hyperalgésie thermique inflammatoire.
- La figure 6 représente l'effet spécifique de l'injection intrathécale d'un ARN interférent dirigé contre l'ARN messager d'ASIC3 sur les niveaux dans les ganglions rachidiens des ARN messagers des canaux ASIC et du canal TRPV1, et l'effet analgésique de cet ARN interférent dans un modèle de douleur inflammatoire.
- La figure 7 représente l'effet de la toxine APETx2 sur l'activité des canaux ASIC3 humains exprimés dans la lignée de neurones sensoriels F11.

### EXEMPLES

### Exemple 1 : matériel et méthode

### Purification de la toxine peptidique APETx2 de l'anémone de mer Anthopleura elegantissima

Un pool de polypeptides a été isolé à partir d'un extrait hydroalcoolique (eau-méthanol) brut de l'anémone de mer *Anthopleura elegantissima* ([réf. 39], précitée) par chromatographie d'échange cationique sur QAE-Sephadex A-25 (4,5x400 nm) éluée avec de l'acétate, d'ammonium (pH 8,3), suivie d'une chromatographie d'exclusion diffusion sur Sephadex G50 (12x140 cm) dans l'acide acétique 1M.

Six fractions ont été testées sur des canaux ASIC3 exprimés dans des ovocytes de Xénope ([réf. 41], précitée). Une fraction, ayant inhibé plus de 80% du courant ASIC3, a été purifiée par chromatographie liquide à haute performance (HPLC) à polarité de phase inversée (Waters Symmetry C18, 4,6x250 mm), avec un gradient linéaire de 10 à 40% de solvant B (acétonitrile / TFA 0,1%) pendant 30 min., à 1 ml/min: La séparation a été réalisée sur un système HP1100 (Hewlett Packard, USA) couplé à un détecteur à réseau de diodes avec un contrôle de l'absorbance UV à 220 et 280 nm.

La fraction peptidique active a alors été purifiée sur une colonne d'échange cationique TSK-SP5PW (7,5x75 mm) (Tosoh, Japon) équilibrée avec un mélange eau / acide acétique 1% en utilisant un gradient linéaire de 0 à 100% d'acétate d'ammonium 1M, pendant 50 min., à 1 ml/min. La purification finale d'APETx2 a été réalisée sur la même colonne HPLC à polarité de phase inversée, en utilisant un gradient linéaire de 20 à 30%, pendant 10 min., suivie par 30-40% de solvant B, pendant 20 min.

Le séquençage de la toxine peptidique APETx2 a été effectué par séquençage N-terminal d'Edman automatisé (477A, Applied Biosystems, USA) après sa réduction avec du 2-mercaptoéthanol et son alkylation avec de la 4-vinylpyridine. La séquence C-terminale du peptide a été confirmée par citraconylation des résidus arginine, suivie d'une digestion par la trypsine. Les fragments tryptiques ont été séparés par HPLC (Waters C18, 2x150 mm) en utilisant un gradient linéaire de 5 à 50% d'acétonitrile / TFA 0,1% dans un mélange eau / TFA 0,1%, pendant 40 min., à 200 µl/min. Les homologies de séquence ont été déterminées à partir du programme BLAST. La détermination de la masse moléculaire a été réalisée par spectrométrie de masse MALDI-TOF sur un système Voyager DE-PRO (Applied Biosystems, USA) dans un mode réflecteur, avec une matrice d'acide α-cyano-4-hydroxycinnamique (Sigma-Aldrich, USA) et une calibration interne. Le spectre de masse a été analysé avec le logiciel Data Explorer, et les masses moléculaires théoriques ont été calculées à partir des données de séquence en utilisant le logiciel GPMAW.

L'arrangement du pont disulfure de la toxine peptidique APETx2 a été déterminé en utilisant le procédé de réduction partielle et de clivage induit par cyanylation ([réf. 42 et 56]).

Un modèle moléculaire de la toxine peptidique APETx2 a été calculé à partir des coordonnées précédemment décrites d'APETx1 ([réf. 40], précitée), en utilisant le logiciel Deep-View Swiss-PDB viewer v3.7. Ce modèle a été optimisé via le serveur Swiss-Model. Les coordonnées BDS-I (1BDS) ont été obtenues à partir de la base de données PDB. Enfin, la structure spatiale de la toxine peptidique APETx2 a été établie par résonance magnétique nucléaire, et consiste essentiellement en un noyau compact lié par des ponts disulfures composé d'un feuillet β à quatre brins ([réf. 43]).

### Culture de la lignée cellulaire F-11 et transfection

La lignée cellulaire F-11 (réf. 44, 45, 46, 47]) a été cultivée sous 5% de CO₂ et à une densité de 50 000 cellules par boîte de Pétri de 35 mm de diamètre. Le milieu de culture contenait du milieu HAM F-12 (Invitogen) supplémenté avec 15% de sérum bovin foetal (ICN Biomedicals), 1 x HAT (hypoxanthine de sodium, aminoptérine et thymidine), 200 µg/ml d'allo-4-hydroxy-L-proline (Sigma-Aldrich), et 1% d'antibiotiques. Un jour après étalement, les cellules ont été transfectées avec l'ADNc d'ASIC1a ou d'ASIC3 (clones de rat) en utilisant la Lipofectamine™ (Invitrogen) selon les instructions du fabricant, à l'aide des vecteurs pCI-ASIC1ar + pIRES₂-EGFP (ratio 1 :2) ou pCI-ASIC3r + pIRES₂-EGFP (ratio 1 :10) ([réf. 36], précitée). Pour les expériences concernant le clone ASIC3 humain (cf expériences sur les effets de la toxine APETx2 sur la forme humaine du canal ASIC3), les cellules ont été transfectées avec l'ADNc d'ASIC3 humain en utilisant l'agent de transfection JetPEI (Polyplus-transfection) selon les instructions du fabricant, à l'aide du vecteur pASIC3h-IRES-EGFP. Les cellules ont été utilisées pour les expériences de Patch-clamp (technique électrophysiologique d'enregistrement de courants ioniques transitant à travers les membranes cellulaires) 2 à 4 jours après transfection.

### Marquage rétrograde d'afférences cutanées

Les neurones du ganglion de la racine dorsale (ganglions rachidiens ou DRG) innervant la peau ont été marqués par injections sous-cutanées de 5 x 1 µl de colorant fluorescent Dil (5% DMSO, Molecular probes) dans les faces dorsales des pattes postérieures de rat. Le colorant a été injecté deux semaines avant le sacrifice des rats afin de réaliser une culture primaire de ganglion de la racine dorsale.

### Culture primaire de neurones des ganglions de la racine dorsale marqués

Les ganglions de la racine dorsale L3-L6 lombaires de rats Wistar (8-11 semaines) ont été disséqués de manière bilatérale et dissociés de manière enzymatique avec 0,1% de collagénase. Les cellules ont alors été étalées sur des boîtes de Pétri recouvertes de collagène de 35 mm de diamètre et maintenues en culture à 37°C (95% air / 5% de CO₂) dans du milieu DMEM contenant 5% de sérum de veau foetal. Les expériences électrophysiologiques ont été réalisées 1 à 8 jours après étalement.

### Electrophysiologie

La configuration cellule entière de la technique de Patch-clamp a été utilisée ([réf. 48]) afin de mesurer les courants membranaires (voltage imposé) ou les potentiels membranaires (courant imposé). Les enregistrements ont été réalisés à température ambiante en utilisant un amplificateur RK-400 (Bio-Logic Science Instruments) avec un filtre passe-bas de 3kHz (Krohn-Hite). Les données ont été échantillonnées à 10 kHz, numérisées à l'aide d'un convertisseur Digidata 1322A A-D/D-A (Axon Instruments) et enregistrées sur un disque dur en utilisant le logiciel pClamp (version 9.2.0.11, Axon Instruments). Les pipettes d'enregistrement (1-4 MOhm) contenaient (en mM) : 135 KCI, 2,5 Na₂-ATP, 2 MgCl₂, 2.1 CaCl₂, 5 EGTA, 10 HEPES (pH 7,25 avec KOH). Diverses solutions tamponnées et drogues d'intérêt ont été appliquées à des cellules transfectées prises individuellement en utilisant un système de microperfusion développé localement et contrôlé par des microélectrovannes (Sirai, Italie) permettant des changements rapides de solution. La solution de bain contrôle contenait (en mM) : 145 NaCl, 5 KCI, 2 MgCl₂, 2 CaCl₂, 10 HEPES (pH 7,4 avec NaOH). Le milieu MES a été utilisé à la place du milieu HEPES pour tamponner la solution à un pH allant de 6 à 5, et les courants ASIC ont été induits par changement rapide d'une solution contrôle à pH 7,4 à une solution test acide à l'aide du système de microperfusion. Pour les expérimentations réalisées sur les neurones DRG, du glucose (10 mM) a été ajouté à la solution de bain contrôle. Des conditions hypertoniques ont été obtenues en ajoutant du mannitol ou du sucrose aux solutions de bain externes, comme indiqué dans le texte.

### Comportement nociceptif chez le rat

Des rats mâles adultes (7-8 semaines) Wistar (Charles River, France) ont été placés dans des cages en plastique avec un nycthémère de 12h (avec la lumière de 8:00 à 20:00) et avec un accès ad libitum à la nourriture et à l'eau. Les rats ont été acclimatés pendant au moins 1 semaine avant les expérimentations. Pour les expériences de comportement, les rats ont été placés dans une chambre d'observation transparente où ils ont été acclimatés pendant 20-30 minutes. Ils ont alors été immobilisés pendant que 20 µl d'une solution saline (0,9% ou 2% NaCl + 20 mM HEPES, 7,4≤pH≤6,6 supplémenté ou non avec 10 µM d'acide. arachidonique et/ou 10µM de toxine APETx2 ou 60 nM de toxine PcTx1), ont été administrés en sous-cutanée dans la face dorsale de la patte postérieure droite en utilisant une aiguille de grosseur 30G connectée à une seringue Hamilton de 100 µl. Le comportement nociceptif (c'est-à-dire le nombre de tressaillements de la patte) a été enregistré sur une période de 5 minutes commençant immédiatement après l'injection ([réf. 59]):

### Hyperalgésie thermique induite par l'inflammation chez les rats

La sensibilité à la chaleur de rats mâles adultes Wistar (Charles River, France) a été testée en mesurant le temps mis par l'animal pour lever une des pattes arrières lorsqu'il est placé sur une plaque à 50°C (Bioseb, France) avant et après l'induction d'une inflammation par injection sous-cutanée d'une solution d'adjuvant complet de Freund (CFA, Sigma-Aldrich, France). Les rats ont été acclimatés à la salle d'expérimentation pendant au moins 30 minutes, et chaque mesure a été réalisée en double. Une première mesure a été réalisée avant l'induction de l'inflammation. Les rats ont alors été anesthésiés (isoflurane) pendant que 150 µl de CFA dilué 1 :1 avec une solution saline (NaCl 0.9%) contenant soit des toxines (PcTx1 ou APETx2, 120 nM et 20 µM, respectivement) soit le véhicule ont été injectés (aiguille de grosseur 26G connectée à une seringue de 1 ml) en sous-cutanée dans la face plantaire de l'une des pattes arrières. Le temps mis par l'animal pour lever la patte arrière injectée a alors été mesurée à 50°C, 2, 4 et 24 heures après l'injection de CFA.

### Injections intrathécales des ARN interférents chez les rats

Un ARNi spécifique des canaux ASIC3 (no.1121; CTACACGCTATGCCAAGGA, SEQ ID NO : 1) et son contrôle (no.1121 S; GCTCACACTACGCAGAGAT, SEQ ID NO : 2) ont été conçus dans le laboratoire et synthétisés par la société MWG Biotech (Allemagne). L'ARNi spécifique d'ASIC3 a été validé par quantification des niveaux d'ARN messagers par RT-PCR quantitative pour les différents canaux ASIC et pour le canal TRPV1, après injections intrathécales chez le rat dans la région lombaire de la moelle épinière, à raison d'une injection par jour pendant 3 jours consécutifs, avant le sacrifice de l'animal. La même procédure a été utilisée avant induction de l'inflammation par le CFA. Chaque injection correspond à un volume de 10 µl, contenant 2 µg d'ARNi mélangé avec l'agent de transfection i-Fect (Neuromics) dans un rapport de 1 pour 4.

### Produits chimiques

Les milieux HEPES (acide 4-(2-hydroxyéthyl)1-pipérazineéthanesulfonique) et MES (acide 2-(N-Morpholino)éthanesulfonique ; C₆H₁₃NO₄S•H₂O), le mannitol, la capsazépine, l'acide arachidonique ont été acquis chez Sigma.

### Analyse des données

Les analyses de données ont été réalisées en utilisant les logiciels Microcal™ Origin 6.0® et GraphPad Prism 4.03. Les données ont été représentées sous forme d'une moyenne ± erreur type de la moyenne, et les différences statistiques entre les jeux de données ont été évaluées en utilisant soit le test t de Student soit l'analyse de variance unidirectionnelle (one-way ANOVA) suivi par des tests *post hoc* lorsque nécessaires.

### Exemple 2 : courant de type ASIC3 dans les neurones des ganglions rachidiens innervant la peau

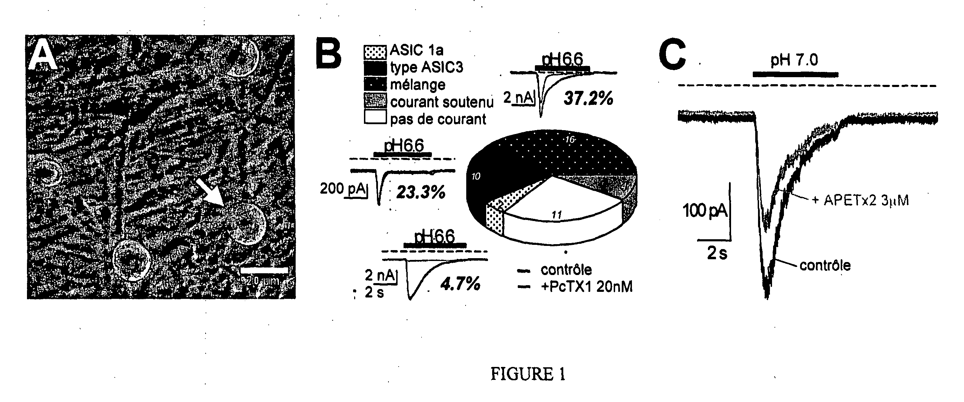

Les courants ASIC activés par des acidifications extracellulaires modérées ont été enregistrés dans les neurones des ganglions rachidiens innervant la peau chez le rat et identifiés par marquage rétrograde à l'aide du colorant fluorescent Dil (figure 1).

Les valeurs de pH modérés utilisées dans ces expérimentations (i.e. pH 6,6 et pH 7,0) ont été choisies afin d'activer principalement les courants de type ASIC1 et ASIC3, du fait que les courants de type ASIC2 et TRPV1 avaient été décrits comme étant activés par une acidification plus drastique ([réf. 49] et [réf. 2], précitée).

La figure 1A montre un neurone cutané des ganglions rachidiens positif au Dil typique (cf. flèche) utilisé pour les expérimentations Patch-clamp en configuration cellule entière. Ces neurones ont un potentiel de membrane de repos de -55.0±1,8 mV et une capacité de membrane de 39,6±1,8 pF (n = 42 et 43 neurones, respectivement, données provenant de quatre cultures différentes).

La figure 1B montre que 65,8 ±6,3% des neurones cutanés des ganglions rachidiens testés (4/7, 8/11, 8/10 et 8/15 données provenant de quatre cultures différentes) présentent un courant de type ASIC transitoire induit par pH 6,6 avec une amplitude moyenne de - 60,3±16,0 pA/pF (n = 28). Parmi les neurones cutanés des ganglions rachidiens restant (34,2±6,3%) l'application externe d'un pH 6,6 induit soit aucun courant (n = 11) soit seulement un petit courant soutenu (-1,2±0,5 pA/pF, n = 4).

Pour distinguer entre les courants de type ASIC induit par pH 6,6 dans les neurones cutanés des ganglions rachidiens, la toxine PcTx1 spécifique d'ASIC1a a été utilisée ([réf. 36], précitée), un inhibiteur sélectif des canaux homomères ASIC1a.

La figure 1B (panneau droit) montre que 4,7% (2/43) de ces neurones montrent un courant largement inhibé par la toxine (inhibition > 90% ; i.e., courant homomère ASICIa), 23,3% (10/43) montrent des courants insensibles à la toxine (inhibition < 10% ; i.e., courants de type ASIC3) et 37,2% (16/43) présentent des courants partiellement inhibés (10%≤inhibition≤90% ; i.e., mélange de courants de type ASIC3 et homomère ASIC1a). La figure 1C confirme la participation d'ASIC3 dans ces courants du fait de leur sensibilité partielle à la toxine peptidique APETx2, qui bloque spécifiquement les canaux contenant l'isoforme ASIC3 ([réf: 38], précitée).

En outre, l'utilisation d'une acidification modérée (pH 6,6) pour induire les courants ASIC dans les neurones cutanés des ganglions rachidiens a permis d'exclure la plupart, sinon tous les courants de type ASIC2a et TRPV1. Le courant ASIC le plus abondant activé par les acidifications modérées dans les neurones cutanés des ganglions rachidiens a été ainsi le courant de type ASIC3 avec un score de 60,5% (26/43).

### Exemple 3 : potentialisation par deux stimuli inflammatoires, pression osmotique et acide arachidonique, de courant ASIC de neurones cutanés des ganglions rachidiens activés par une acidification modérée

Dans des tissus endommagés ou enflammés, plusieurs médiateurs potentiels se rencontrent dans le fluide interstitiel et forment un exsudat inflammatoire ([réf. 50]) dont le contenu est acide et hyperosmotique ([réf. 51]). Ainsi l'effet de l'hyperosmolarité sur des courants ASIC de neurones cutanés des ganglions rachidiens activés par une acidification modérée a été étudié.

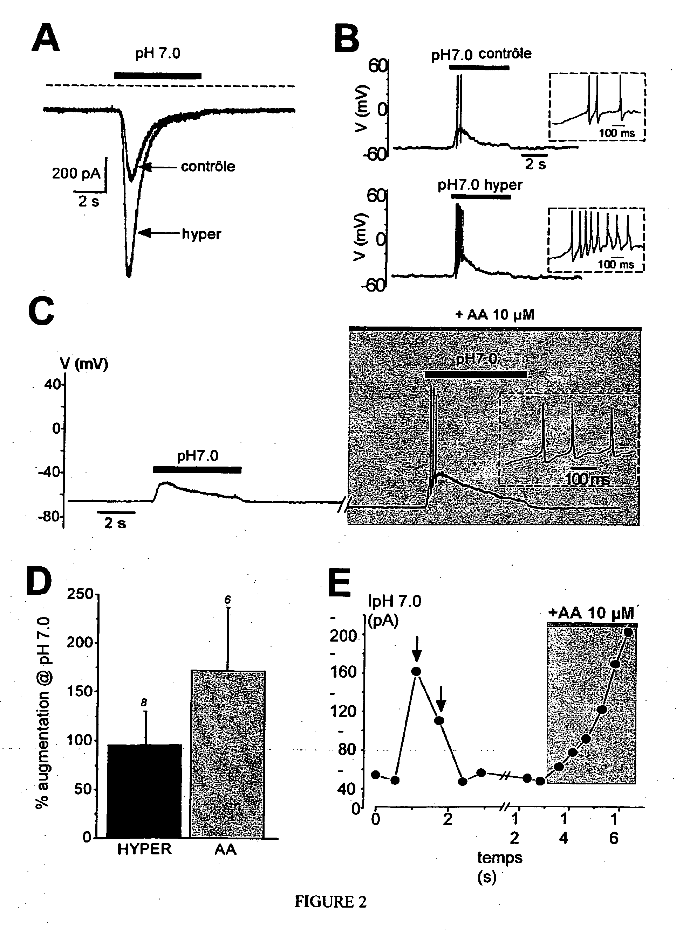

Les figures 2A, 2D et 2E montrent que l'hyperosmolarité, co-appliquée avec une acidification modérée externe (pH 7,0, cf. flèches dans la figure 2E), est capable d'augmenter fortement le courant de type ASIC induit par pH 7,0 dans ces neurones (augmentation de 95%±35%, n = 8, P<0,01, test t de Student par paires) conduisant à une augmentation de l'excitabilité neuronale en provoquant plus de potentiels d'action (figure 2B).

Un effet positif du médiateur inflammatoire, acide arachidonique (AA), sur les courants ASIC a déjà été décrit ([réf. 52 et 53]). Cet effet a été confirmé sur le courant ASIC induit par pH 7,0 natif à partir de neurones cutanés des ganglions rachidiens (figure 2D ; augmentation par 172±65%, n = 6, P = 0,06, test t de Student par paires). De manière importante, la figure 2C montre que cet effet de l'acide arachidonique a également conduit à une excitabilité accrue des neurones cutanés des ganglions rachidiens par augmentation du déclenchement du potentiel d'action.

Les cinétiques de ces deux effets sont différentes. En effet, la figure 2E montre qu'alors que la potentialisation induite par l'hyperosmolarité du courant ASIC natif est immédiate (co-application avec le saut de pH), l'effet de l'acide arachidonique sur le courant ASIC a besoin de quelques minutes pour être pleinement opérationnel. Par ailleurs, les applications répétées d'hyperosmolarité sur la même cellule conduisent à une diminution de l'effet (cf. flèches) alors que l'effet de l'acide arachidonique augmente à un maximum aussi longtemps qu'il est appliqué à une cellule (cadre grisé). Ainsi, pris ensemble, ces résultats montrent que (i) les courants ASIC activés par une acidification modérée (pH 7,0) dans les neurones cutanés des ganglions rachidiens sont suffisamment importants pour amener le potentiel de membrane au seuil de déclenchement des potentiels d'action, (ii) deux signaux inflammatoires tels que l'hypertonicité et l'acide arachidonique augmentent l'excitabilité neuronale à travers un effet potentialisateur sur le courant ASIC activé par des acidifications modérées.

### Exemple 4: sensibilité du canal ASIC3 recombinant à la pression osmotique dans des cellules F-11 dérivées de ganglions rachidiens

Afin d'analyser plus précisément l'effet de la pression osmotique sur les différentes isoformes d'ASIC, les canaux ASIC1a et ASIC3 ont été exprimés de manière hétérologue dans la lignée cellulaire F-11 ([réf. 44, 45, 46, 47], précitées).

Les canaux ASIC1a et ASIC3 ont été utilisés car (i) ils sont représentatifs des courants ASIC exprimés dans les neurones des ganglions rachidiens de rat (réf. 54] et [réf. 55], précitée) (cf. également figure 1), et (ii) leur pH liminal pour l'activation est proche du pH 7,0 ([réf. 2], précitée).

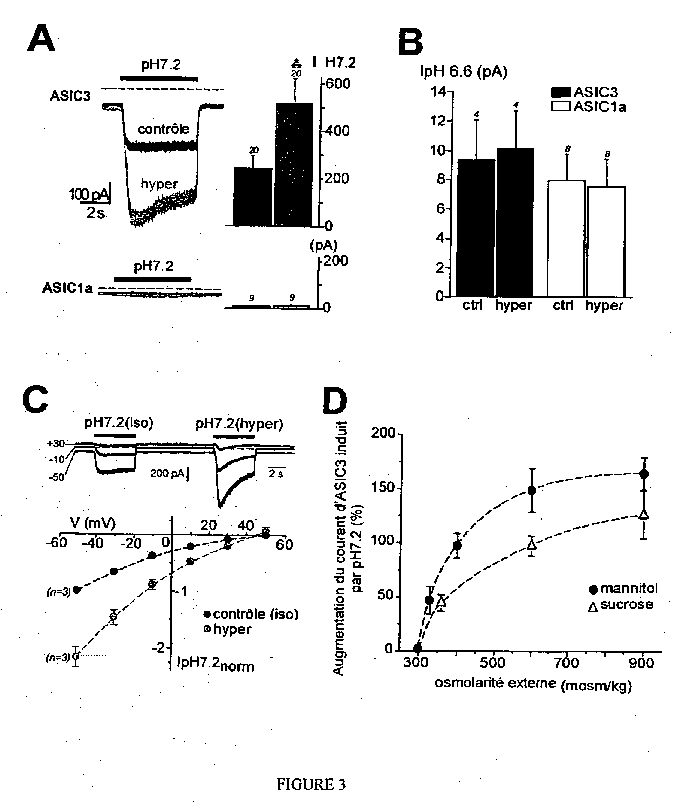

La figure 3A montre que l'hyperosmolarité (600 mosmol.kg⁻¹ avec du mannitol) est capable de potentialiser significativement le courant ASIC3 activé par pH 7,2 (augmentation de 148±20%, n = 20, P<0,001, test t de Student par paires). En revanche, l'acidification externe à pH 7,2 ne produit aucun courant ASIC1a significatif à partir des cellules transfectées par ASIC1a, et l'hyperosmolarité est sans effet. La figure 3B montre de manière intéressante que l'hyperosmolarité (600 mosmol.kg⁻¹), qui est encore sans effet sur le courant ASIC1a activé par pH 6,6, ne réussit pas à potentialiser le courant ASIC3 activé par pH 6,6, suggérant un effet sur le courant de fenêtre ASIC3 non-inactivant, persistant ([réf. 19], précitée).

Afin de confirmer que les chocs hyperosmotiques potentialisent le courant ASIC3, les courbes I/V du courant induit par pH 7,2 enregistrées à partir de cellules F-11 transfectées ont alors été établies (figure 3C, panneau supérieur). Les courants induits par pH 7,2 contrôle (mesure en condition isotonique) et augmenté (mesure en condition hypertonique) ont le même potentiel d'inversion (49,4±0,7 mV et 45,9±3,4 mV, respectivement, P = 0,48, test t de Student par paires) (figure 3C, panneau inférieur) indiquant que l'hyperosmolarité potentialise réellement le courant ASIC3.

La figure 3D montre que le pourcentage d'augmentation du courant ASIC3 induit par pH 7,2 est presque maximal lorsque l'osmolarité externe atteint 600 mosmol.kg⁻¹. Par ailleurs, l'hypertonicité est le facteur primaire dans cet effet dans la mesure où il est également observé, bien que d'amplitude légèrement inférieure, lorsque les solutions externes hyperosmotiques ont été faites à l'aide de sucrose (figure 3D).

Ces résultats indiquent que l'hyperosmolarité potentialise le courant ASIC3 dans une gamme de pH modérée (i.e. proche de pH 7,2) probablement à travers un effet sur le courant de fenêtre ASIC3.

### Exemple 5: potentialisation du courant de fenêtre ASIC3 non-inactivant par l'acide arachidonique

L'effet de l'acide arachidonique (AA) a été analysé sur des cellules F-11 transfectées par ASIC1a et ASIC3 afin d'examiner l'effet potentialisateur de l'acide arachidonique (AA) sur les courants de type ASIC.

Un effet de l'acide arachidonique sur l'activité des canaux ASICs a déjà été décrit ([réf. 52] et [réf. 53], précitées), mais le mécanisme de l'effet reste peu compris.

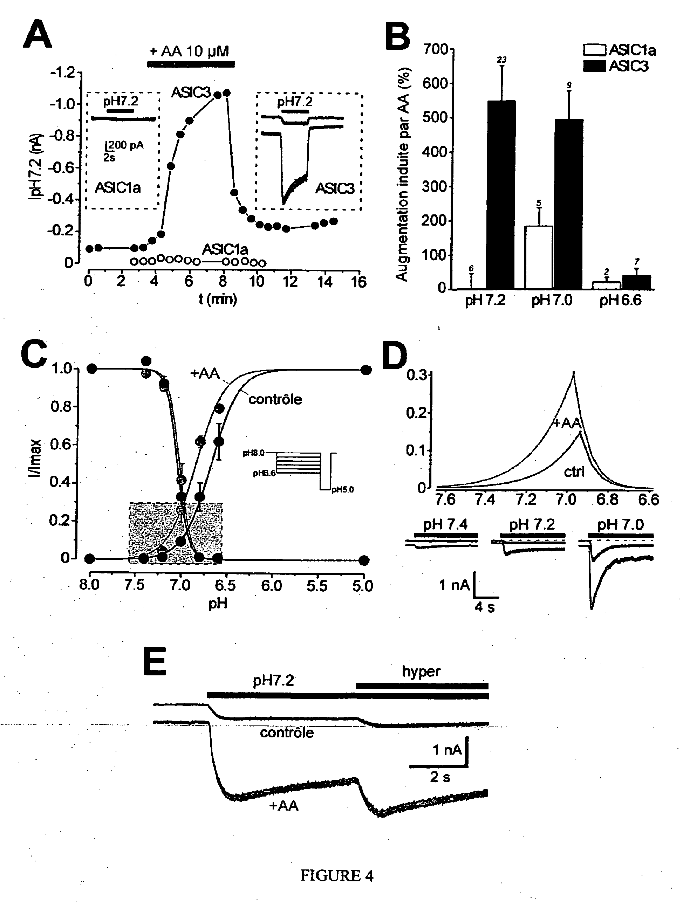

La figure 4A montre que l'acide arachidonique produit une augmentation réversible drastique du courant ASIC3 activé par pH 7,2 alors qu'il n'a pas d'effet sur ASIC1a au même pH. L'acide arachidonique potentialise fortement les courants ASIC3 activés par pH 7,2 et pH 7,0 (augmentation de 547±103%. P<0,0001, n = 23 et 493±84%, P<0,0001, n = 9, respectivement, test de Wilcoxon), alors que le courant ASIC3 activé par pH 6,6 est seulement faiblement augmenté (+40±22%, n = 7, P=0,45, test t de Student par paires) (cf. figure 4B). De manière cohérente avec les précédents résultats ([réf. 53], précitée), l'acide arachidonique augmente également le courant ASIC1a à pH 7,0 (+183±54%, n = 5, P=0,06, test de Wilcoxon) et dans une moindre mesure à pH 6,6 (+21±15%, n = 2), mais cet effet reste beaucoup plus faible que pour ASIC3 (figure 4B).

La figure 4C montre que l'effet puissant de l'acide arachidonique sur le courant ASIC3 résulte d'un changement de la dépendance au pH de l'activation vers des valeurs plus physiologiques alors qu'aucun effet significatif n'est observé sur la courbe d'inactivation dépendante du pH.

En conséquence, le courant de fenêtre ASIC3 non-inactivant est fortement augmenté en présence d'acide arachidonique (figure 4D, panneau supérieur), conduisant à l'activation du canal à des valeurs de pH proches du pH physiologique de repos (i.e., pH 7,4) (figure 4D, panneau inférieur).

Ces résultats indiquent que l'acide arachidonique potentialise préférentiellement les courants ASIC3 activés par une acidité modérée à travers un puissant effet sur le courant de fenêtre non-inactivant. De manière très importante, la figure 4E montre que cet effet de l'acide arachidonique est additif avec l'effet de l'hypertonicité sur le même courant ASIC3. Ce résultat suggère fortement qu'ASIC3 peut intégrer des signaux inflammatoires différents tels que l'acidification modérée, l'hypertonicité et l'acide arachidonique.

### Exemple 6 : contribution d'ASIC3 à la douleur cutanée induite par une acidification modérée

La forte expression d'ASIC3 dans les neurones cutanés des ganglions rachidiens et sa modulation à pH modéré par des stimuli inflammatoires, tel que l'hyperosmolarité et l'acide arachidonique, a conduit à rechercher le rôle d'ASIC3 dans la douleur acide cutanée en conditions normales et inflammatoires.

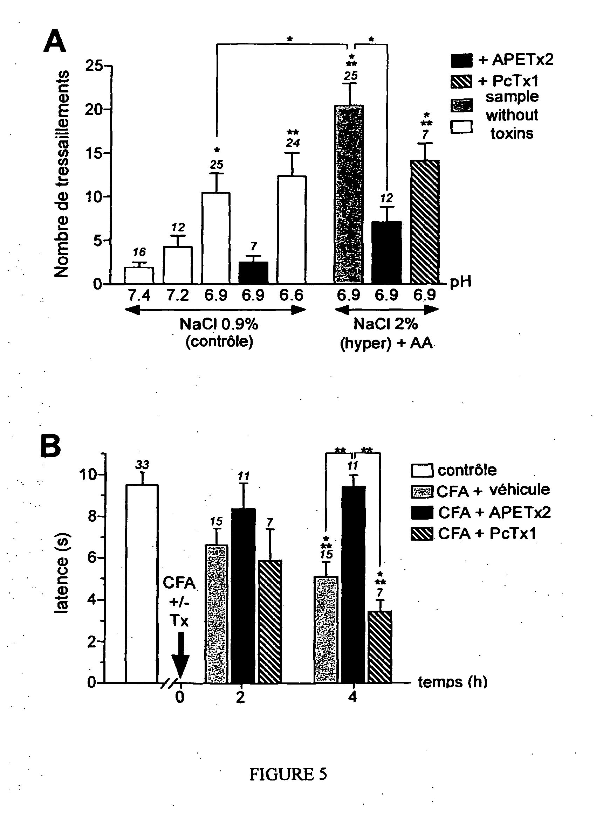

La figure 5A montre le comportement des rats à la douleur suite à l'injection en sous-cutanée de solutions modérément acides (pH 7,4, pH 7,2, pH 6,9, et pH 6,6) dans l'une des pattes arrières. Un comportement à la douleur significatif apparaît à pH 6,9 (score de tressaillement augmente de 2,11±0,67 à pH 7,4 à 11,11±2,54 à pH 6,9, n = 14 et 22, respectivement, P<0,05, test de Kruskal-Wallis suivi post hoc par un test de Dunn). Ce comportement douloureux est aboli en présence de toxine APETx2 mettant ainsi en évidence l'implication d'ASIC3 dans la détection de la douleur acide sous-cutanée.

Dans la mesure où l'hypertonicité et l'acide arachidonique apparaissent comme de forts activateurs synergiques du canal ASIC3 à pH modéré à la fois dans les neurones cutanés des ganglions rachidiens et dans les cellules F-11 exprimant des canaux recombinants (cf. figures 2, 3 et 4), ces signaux inflammatoires ont été co-injectés (NaCl 2%, -600 mOsm.kg⁻¹ et AA 10 mM) ensemble avec un pH modéré (figure 5A). La combinaison de ces trois importants éléments du cocktail inflammatoire augmente le score de tressaillement des rats par rapport au pH 6,9 seul (de 11,1±2,54, n = 22 à 20,42±2,53, n = 25, P<0,05, test de Kruskal-Wallis suivi post hoc par un test de Dunn) (figure 5A).

Ce comportement à la douleur acide a été fortement diminué par la toxine peptidique APETx2, l'inhibiteur spécifique d'ASIC3 ([réf. 38], précitée), alors que la toxine PcTx1 inhibiteur spécifique d'ASIC1a n'a aucun effet significatif (figure 5A).

Ces résultats pris ensemble, suggèrent fortement qu'ASIC3 est le principal capteur de la douleur cutanée induite par une acidification modérée et qu'il participe à la douleur inflammatoire chez le rat.

### Exemple 7 : contribution d'ASIC3 au développement de l'hyperalgésie thermique induite par l'inflammation provoquée par l'adjuvant complet de Freud (CFA) chez le rat

Les effets des toxines peptidiques APETx2 (inhibiteur des canaux contenant l'isoforme ASIC3) et PcTx1 (inhibiteur des canaux homomères d'ASIC1a) ont été testés dans un modèle de douleur cutanée (hyperalgésie thermique induite par CFA) chez le rat afin de confirmer le rôle spécifique d'ASIC3 dans la douleur inflammatoire.

La figure 5B montre que quatre heures après l'induction de l'inflammation par injection de CFA dans une patte arrière, une hyperalgésie thermique significative apparaît.

En revanche, l'hyperalgésie thermique ne se développe pas lorsque la toxine peptidique APETx2 est co-injectée avec le CFA, alors que la toxine PcTx1 n'a aucun effet significatif.

Vingt heures après l'injection de CFA, les animaux traités avec la toxine peptidique APETx2 ne présentent pas un comportement différent de celui des animaux contrôle injectés avec le véhicule au regard de l'hyperalgésie thermique (données non représentées), illustrant probablement une disparition progressive de l'effet de cette toxine au cours du temps.

Ces résultats indiquent qu'ASIC3, mais pas AsIC1a, joue un rôle important dans le ressenti de la douleur inflammatoire au niveau périphérique chez le rat.

### Exemple 8 : Effet inhibiteur spécifique de la toxine APETx2 in vivo

La démonstration de la spécificité de l'inhibition des canaux ASIC3 par la toxine APETx2 au niveau cellulaire (sur des neurones en culture et sur des lignées cellulaires F-11 transfectées), a conduit à rechercher la preuve que les effets analgésiques d'APETx2 observés chez le rat résultaient exclusivement d'une inhibition de l'activité des canaux de type ASIC3. Pour ce faire, une approche d'inhibition génétique des canaux ASIC3 a été réalisée, consistant à observer l'effet d'injections intrathécales d'ARN interférents (ARNi) dirigés spécifiquement contre les canaux ASIC3 chez des rats.

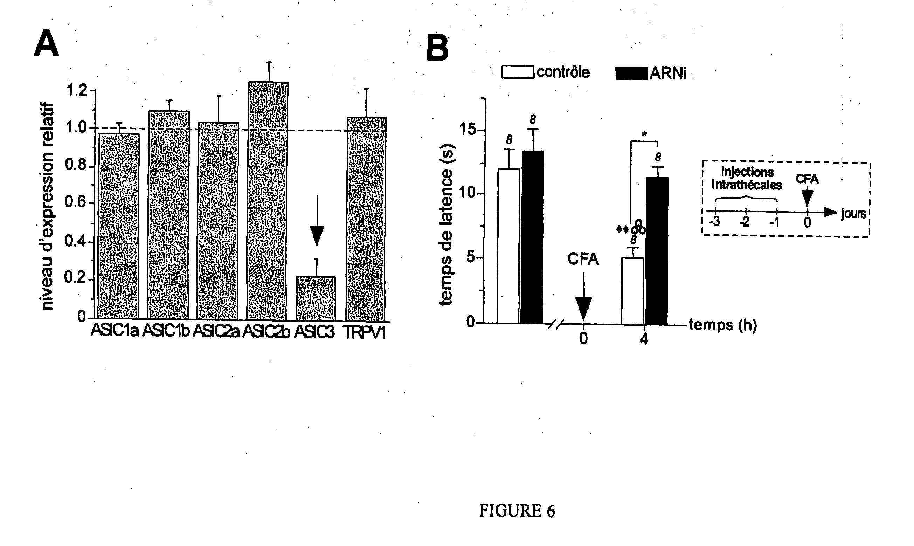

La figure 6A montre l'effet d'injections intrathécales d'ARNi spécifiques des canaux ASIC3 sur les niveaux d'expression de différents canaux ioniques dans les neurones sensoriels de rat qui innervent les pattes postérieures. Les résultats montrent que les injections intrathécales d'ARNi spécifiques des canaux ASIC3 permettent de diminuer fortement et exclusivement l'expression des canaux ASIC3 dans les neurones sensoriels des rats (cf. flèche noire).

La figure 6B montre que les rats soumis à des injections intrathécales d'ARNi spécifiques des canaux ASIC3 pendant les 3 jours précédant l'induction d'une inflammation provoquée par l'adjuvant complet de Freud (CFA) (cf. insert), ne développent pas d'hyperalgésie thermique inflammatoire (ARNi, colonne noire) comparativement à des rats contrôle traités avec des ARNi interférents de composition identique mais de séquence aléatoire (contrôle, colonne blanche). Ce résultat est identique à celui obtenu avec des animaux traités avec la toxine APETx2 (cf. exemple 7).

Ces résultats apportent la preuve que les canaux ASIC3 sont des médiateurs de la douleur inflammatoire, et que les effets analgésiques de la toxine APETx2 résultent exclusivement du blocage de ces canaux chez l'animal.

### Exemple 9 : effet inhibiteur de la toxine APETx2 sur le clone ASIC3 humain

L'effet inhibiteur de la toxine peptidique APETx2 a été testé sur les canaux ASIC3 humains. Pour ce faire, l'amplitude des courants ASIC3 humain a été enregistrée selon la technique de Patch-clamp décrite ci-dessus, suite à des acidifications du milieu extracellulaire (pH8,0 à pH7,0, cf. double flèche), en l'absence ou présence de la toxine APETx2 à 1 µM, à partir de la lignée de neurones sensoriels F-11 transfectée avec le clone ASIC3 humain selon la méthode décrite ci-dessus.

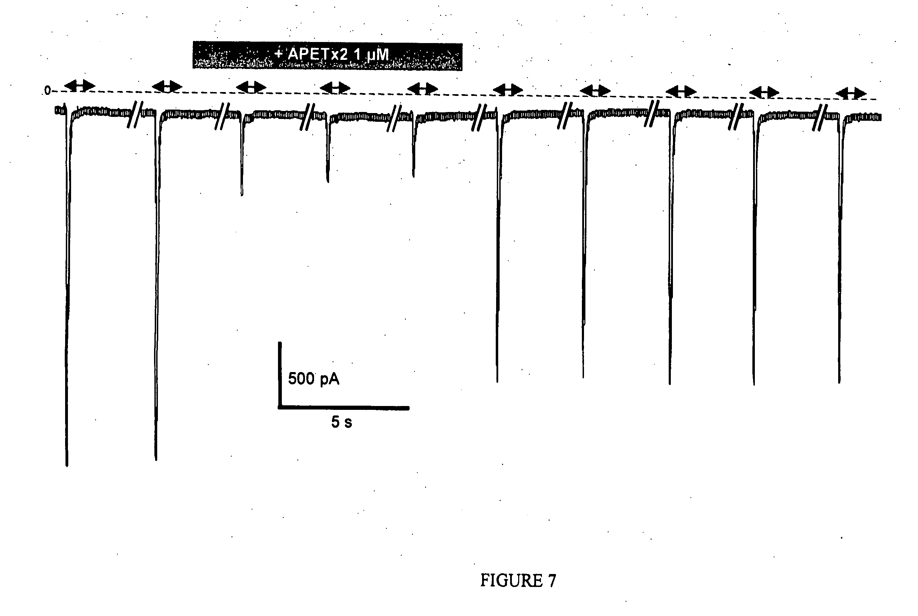

La figure 7 montre que l'amplitude des courants issus des canaux ASIC3 humains est significativement diminuée en présence de la toxine APETx2 (3^{ème} au 5^{ème} pics en partant de la gauche).

Ces résultats indiquent bien que la toxine APETx2 est capable d'inhiber l'activité des canaux ASIC3 humains, et valident l'utilisation potentielle de la toxine APETx2 et de ses dérivés en tant que nouveau composé anti-douleur chez l'homme.

### LISTES DES RÉFÉRENCES

- [ref. 1]: Wemmie et al., Trends Neurosci., 29 : 578-586, 2006
- [ref. 2]: Lingueglia, J. Biol. Chem., 282 : 17325-17329, 2007
- [ref. 3]: Jasti et al., Nature, 449 : 316-323, 2007
- [ref. 4]: Lingueglia et al., J. Biol. Chem., 272 : 29778-29783, 1997
- [ref. 5]: Benson et al., Proc. Natl. Acad. Sci. U.S.A., 95 : 10240-10245,2002
- [ref. 6]: Hesselager et al., J. Biol. Chem., 279 : 11006-11015, 2004
- [ref. 7]: Waldmann et al., J. Biol. Chem., 272 : 20975-20978, 1997a
- [ref. 8]: Bassler et al., J. Biol. Chem., 276 : 33782-33787, 2001
- [ref. 9]: Reeh & Steen, Prog. Brain Res., 113 : 143-151, 1996
- [ref. 10]: Woo et al., Anesthesiology, 101 : 468-475, 2004
- [ref. 11]: Steen et al., Neurosci. Lett., 199 : 29-32, 1995a
- [ref. 12]: Issberner et al., Neurosci. Lett., 208 : 191-194, 1996
- [ref. 13]: Ugawa et al., J. Clin. Invest., 110 : 1185-1190, 2002
- [ref. 14]: Jones et al., J. Neurosci., 24 : 10974-10979, 2004
- [ref. 15]: Waldmann et al., Nature, 386 : 173-177, 1997b
- [ref. 16]: Dube et al., Pain, 117 : 88-96, 2005
- [ref. 17]: Voilley et al., J. Neurosci., 21 : 8026-8033, 2001
- [ref. 18]: Molliver et al., Mol. Pain, 1 : 35, 2005
- [ref. 19]: Yagi et al., Circ. Res:, 99 : 501-509, 2006
- [ref. 20]: Sutherland et al., Proc. Nat. Acad. Sci. USA, 98 : 711-716, 2001
- [ref. 21]: Price et al., Neuron, 32 : 1071-1083, 2001
- [ref. 22]: Sluka et al., Pain, 106 : 229-239, 2003
- [ref. 23]: Sluka et al., Pain, 129 : 102-112, 2007
- [ref. 24]: Xie et al., J. Neurophysiol., 87 : 2835-2843, 2002
- [ref. 25]: Chen et al., Proc. Natl. Acad. Sci. U.S.A., 99 : 8992-8997, 2002
- [ref. 26]: Babinski et al., J. Biol. Chem., 275 : 28519-28525, 2000
- [ref. 27]: Askwith et al., Neuron, 26 : 133-141, 2000
- [ref. 28]: Moczydlowski et al., J. Membr. Biol., 105 : 95-111, 1988
- [ref. 29]: Norton, Toxicon, 29 : 1051-1084, 1991
- [ref. 30]: Harvey et al., Ann. NY Acad. Sci., 710 : 1-10, 1994
- [ref. 31]: Uchitel, Toxicon, 35 : 1161-1191, 1997
- [ref. 32]: Tytgat et al., Trends Pharmacol. Sci., 20 : 444-447, 1999
- [ref. 33]: Escoubas et al., Biochimie, 82 : 893-907, 2000b
- [ref. 34]: Hugues et al., Proc. Nat. Acad. Sci. USA, 79 : 1308-1312, 1982
- [ref. 35]: Shakkottai et al., J. Biol. Chem., 276 : 43145-43151, 2001
- [ref. 36]: Escoubas et al., J. Biol. Chem., 275(33) : 25116-25121, 2000
- [ref . 37]: Mazzuca et al., Nature Neurosci., 10(8) : 943-945, 2007
- [ref. 38]: Diochot et al., Embo J., 23 : 1516-1525, 2004
- [ref. 39]: Bruhn et al., Toxicon, 39 : 693-702, 2001
- [ref. 40]: Diochot et al., Mol. Pharmacol., 64 : 59-69, 2003
- [ref. 41]: Diochot et al., J. Biol. Chem., 273 : 6744-6749, 1998
- [ref. 42]: Wu et al., Anal. Biochem., 235 : 161-174, 1996
- [ref .43]: Chagot et al., Protein Sci., 14 : 2003-2010, 2005
- [ref. 44]: Platika et al., Proc. Natl. Acad. Sci. U.S.A., 82 : 3499-3503, 1985
- [ref. 45]: Francel et al., J. Neurochem., 48 : 1624-1631, 1987
- [ref. 46]: Deval et al., J. Biol. Chem., 281 : 1796-1807, 2006
- [ref.: 47] Ettaiche et al., J. Neurosci., 26 : 5800-5809, 2006
- [ref. 48]: Hamill et al., Pflugers Arch., 391 : 85-100, 1981
- [ref. 49]: Tominaga et al., Neuron, 21 : 531-543, 1998
- [ref. 50]: Steen et al., J. Neurosci., 15 : 3982-3989, 1995b
- [ref. 51]: Vakili et al., Surg. Forum, 21 : 227-228,1970
- [ref. 52]: Allen & Attwell, J. Physiol., 543 : 521-529, 2002
- [ref. 53]: Smith et al., Neuroscience, 145 : 686-698, 2007
- [ref. 54]: Mamet et al., J. Neurosci., 22 : 10662-10670, 2002
- [ref. 55]: Deval et al., Neuropharmacology, 44 : 662-671, 2003
- [ref. 56]: Qi et al., Biochemistry, 40 : 4531-4538, 2001
- [ref. 57]: Chen et al., Proc. Natl. Acad. Sci. U.S.A., 95 : 10240-10245, 1998
- [ref. 58]: Bode et al., Nature, 409 : 35-36, 2001
- [ref. 59]: Alessandri-Haber et al., Pain, 118 : 70-79, 2005
- [ref. 60]: Ikeuchi et al., J. Pain, 10:336-342, 2009
- [ref. 61]: Ständer et Schmelz, Eur. J. Pain, 10 : 473-478

## Revendications

1. Toxine peptidique APETx2 de l'anémone de mer *Anthopleura elegantissima,* peptides isolés d'autres venins d'anémone de mer ou d'autres espèces marines de la même famille présentant de 60 à 99% d'identité de séquence avec la séquence de 42 acides aminés de la toxine peptidique APETx2, et peptides isolés du venin de l'anémone de mer *Anthopleura elegantissima* présentant (i) au moins une substitution sur les positions 3, 5, 8, 9, 10, 15-17, 23, 31-33, 36, 39 et 41 ou (ii) une extension d'au moins un acide aminé sur les extrémités N- et/ou C-terminales, de la séquence de 42 acides aminés de la toxine peptidique APETX2, pour une utilisation comme médicament analgésique,
lesdits peptides conservant la propriété de la toxine peptidique APETx2 à inhiber les canaux de type ASIC3.

2. Toxine peptidique APETx2 et peptides pour une utilisation selon la revendication 1, dans la prévention ou le traitement de la douleur induite par une activation des canaux de type ASIC3.

3. Toxine peptidique APETx2 et peptides pour une utilisation selon la revendication 2, dans laquelle la douleur résulte d'une inflammation.

4. Toxine peptidique APETx2 et peptides pour une utilisation selon la revendication 2, dans laquelle la douleur résulte d'une acidose tissulaire.

5. Toxine peptidique APETx2 et peptides pour une utilisation selon la revendication 4, dans laquelle les situations douloureuses sont choisies dans le groupe constitué par les ischémies, les fractures, les hématomes, les oedèmes, les phlyctènes, les infections locales, les lésions tissulaires y compris les incisions liées à un acte chirurgical, les blessures oculaires et les tumeurs y compris les tumeurs et métastases osseuses.

6. Toxine peptidique APETx2 et peptides pour une utilisation selon la revendication 1, dans la prévention ou le traitement des démangeaisons.

7. Toxine peptidique APETx2 et peptides pour une utilisation selon l'une quelconque des revendications 1 à 6, dans laquelle ledit médicament est/administré par voie périphérique.

8. Toxine peptidique APETx2 et peptides pour une utilisation selon la revendication 7, dans laquelle ledit médicament est destiné à être administré par voie sous-cutanée.

9. Toxine peptidique APETx2 et peptides pour une utilisation selon la revendication 7, dans laquelle ledit médicament est destiné à être administré par voie intramusculaire.

10. Toxine peptidique APETx2 et peptides pour une utilisation selon la revendication 7, dans laquelle ledit médicament est destiné à être administré par voie transcutanée.

11. Toxine peptidique APETx2 et peptides pour une utilisation selon la revendication 7, dans laquelle ledit médicament est destiné à être administré par voie cutanée.

## Claims

1. APETx2 peptide toxin from the *Anthopleura elegantissima* sea anemone, peptides isolated from other sea anemone venoms or from other marine species of the same family having 60 to 99% sequence identity with the sequence of 42 amino acids of the APETx2 peptide toxin, and peptides isolated from the venom of the *Anthopleura elegentissima* sea anemone having (I) at least a substitution at the 3, 5, 8, 9, 10, 15-17, 23, 31-33, 36, 39 and 41 positions or (ii) an extension of at least one amino acid at the N-terminal and/or C-terminal ends, of the sequence of 42 amino acids of the APETx2 peptide toxin, for use as an analgesic drug,
said peptides presenting the same property of the APETx2 peptide toxin of Inhibiting ASIC3-like channels.

2. APETx2 peptide toxin and peptides for use according to claim 1, for the prevention or treatment of pain induced by the activation of the ASIC3-like channels.

3. APETx2 peptide toxin and peptides for use according to claim 2, wherein pain results from Inflammation.

4. APETx2 peptide toxin and peptides for use according to claim 2, wherein pain results from tissue acidosis.

5. APETx2 peptide toxin and peptides for use according to claim 4, wherein painful situations are chosen from among the group composed of ischaemias, fractures, haematomas, oedemas, phlyctenae, local infections, tissue lesions including incisions related to a surgical procedure, eye injuries and tumours including bone tumours and metastases.

6. APETx2 peptide toxin and peptides for use according to claim 1, for the prevention or treatment of itching.

7. APETx2 peptide toxin and peptides for use according to any one of claims 1 to 6, wherein said drug is intended to be administered via the peripheral route.

8. APETx2 peptide toxin and peptides for use according to claim 7, wherein said drug is intended to be administered via the subcutaneous route.

9. APETx2 peptide toxin and peptides for use according to claim 7, wherein said drug is intended to be administered via the intramuscular route.

10. APETx2 peptide toxin and peptides for use according to claim 7, wherein said drug is intended to be administered via the transdermal route.

11. APETx2 peptide toxin and peptides for use according to claim 7, wherein said drug is intended to be administered via the cutaneous route.

## Patentansprüche

1. Peptidtoxin APETx2 der Meeresanemone Anthopleura elegantissima, isolierte Peptide aus anderen Giften der Meeresanemone oder anderen marinen Arten derselben Familie, die 60 bis 99 % Sequenzidentität mit der Sequenz von 42 Aminosäuren des Peptidtoxins APETx2 aufweisen, sowie isolierte Peptide aus dem Gift der Meeresanemone Anthopleura elegantissima, die (i) mindestens eine Substitution an den Positionen 3, 5, 8, 9, 10, 15-17, 23, 31-33, 36, 39 und 41 oder (ii) eine Verlängerung um mindestens eine Aminosäure an den N- und/oder C-terminalen Enden der Sequenz von 42 Aminosäuren des Peptidtoxins APETx2 aufweisen, zur Verwendung als schmerzstillendes Arzneimittel,
wobei die Peptide die Eigenschaft des Peptidtoxins APETx2 bewahren, Kanäle vom Typ ASIC3 zu hemmen.

2. Peptidtoxin APETx2 und Peptide zur Verwendung nach Anspruch 1, zur Vorbeugung oder Behandlung eines Schmerzzustandes, der durch eine Aktivierung von Kanälen des Typs ASIC3 induziert wird.

3. Peptidtoxin APETx2 und Peptide zur Verwendung nach Anspruch 2, wobei der Schmerzzustand von einer Entzündung herrührt.

4. Peptidtoxin APETx2 und Peptide zur Verwendung nach Anspruch 2, wobei der Schmerzzustand von einer Gewebeazidose herrührt.

5. Peptidtoxin APETx2 und Peptide zur Verwendung nach Anspruch 4, wobei die Schmerzzustände ausgewählt sind aus der Gruppe bestehend aus Ischämien, Frakturen, Hämatomen, Ödemen, Phlyctenen, lokalen Infektionen, Gewebsläsionen einschließlich Schnitten von chirurgischen Eingriffen, Augenverletzungen sowie Tumoren einschließlich Knochentumoren und Knochenmetastasen.

6. Peptidtoxin APETx2 und Peptide zur Verwendung nach Anspruch 1, zur Vorbeugung oder Behandlung von Hautjucken.

7. Peptidtoxin APETx2 und Peptide zur Verwendung nach einem der Ansprüche 1 bis 6, wobei das Arzneimittel zur Verabreichung auf peripherem Weg vorgesehen ist.

8. Peptidtoxin APETx2 und Peptide zur Verwendung nach Anspruch 7, wobei das Arzneimittel zur Verabreichung auf subkutanem Weg vorgesehen ist.

9. Peptidtoxin APETx2 und Peptide zur Verwendung nach Anspruch 7, wobei das Arzneimittel zur Verabreichung auf intramuskulärem Weg vorgesehen ist.

10. Peptidtoxin APETx2 und Peptide zur Verwendung nach Anspruch 7, wobei das Arzneimittel zur Verabreichung auf transkutanem Weg vorgesehen ist.

11. Peptidtoxin APETx2 und Peptide zur Verwendung nach Anspruch 7, wobei das Arzneimittel zur Verabreichung auf kutanem Weg vorgesehen ist.
